# EUROPÄISCHE PATENTSCHRIFT

(11) **EP 0 966 307 B1**
(45) Veröffentlichungstag und Bekanntmachung des Hinweises auf die Patenterteilung: **04.09.2002**
(21) Anmeldenummer: 98912435.9
(22) Anmeldetag: 04.03.1998
(51) Int. Cl.: A61L 2/18

(54) **DESINFEKTIONSVERFAHREN**
DISINFECTION METHOD
PROCEDE DE DESINFECTION

(30) Priorität: 13.03.1997 DE 19710256
(43) Veröffentlichungstag der Anmeldung: 29.12.1999
(73) Patentinhaber: Ecolab GmbH & Co. OHG, 40589 Düsseldorf (DE)
(72) Erfinder: DISCH, Karlheinz, D-42781 Haan (DE); BANSEMIR, Klaus-Peter, D-40764 Langenfeld (DE); HACHMANN, Klaus, D-40723 Hilden (DE)
(86) Internationale Anmeldenummer: EP9801193
(87) Internationale Veröffentlichungsnummer: WO98040109

(56) Entgegenhaltungen:
- DE-A- 4 200 066
- US-A- 5 043 357
- US-A- 5 178 830

## Beschreibung

Gegenstand der im folgenden beschriebenen Erfindung ist ein chemothermisches Desinfektionsverfahren für medizinische Instrumente und Geräte, insbesondere für Hämodialysegeräte.

Bei den Desinfektionsverfahren für medizinische Instrumente und Geräte unterscheidet man in erster Linie danach, ob die Desinfektion durch chemische Wirkstoffe und/oder erhöhte Temperatur vorgenommen wird. Die rein thermische Desinfektion kommt für viele Instrumente und insbesondere für kompliziert aufgebaute Geräte meist nicht in Frage, da viele der verwendeten Materialien gegen die notwendigen hohen Temperaturen nicht beständig sind. Die rein chemische Desinfektion erfordert hochwirksame Mikrobizide, wie etwa Aldehyde, Percarbonsäuren oder Hypochlorit, die in der Handhabung problematisch sind und/oder in ihrem Rückstandsverhalten zu Beschwerden.Anlaß geben können. Da die desinfizierende Wirkung der meisten Wirkstoffe mit steigender Temperatur zunimmt, können bei den chemothermischen Verfahren zwar auch weniger aggressive Mikrobizide eingesetzt werden; andererseits steigt mit zunehmender Temperatur die Korrosionsneigung bei den meisten Materialien an. Trotz vieler Vorschläge auch zur chemothermischen Desinfektion von medizinischen Instrumenten und Geräten ist man daher auch heute noch bemüht, insgesamt anwendungstechnisch besser geeignete Verfahren zu finden. Eine der jüngeren Entwicklungen stellt hier das in der deutschen Offenlegungsschrift 42 00 066 vorgeschlagene Verfahren dar, bei dem die Instrumente bei mehr oder weniger erhöhter Temperatur mit wäßrigen Lösungen von Zitronensäure, die gegebenenfalls auch Äpfelsäure und Milchsäure enthalten können, behandelt werden.

Die hier beschriebenen wäßrigen Desinfektionsmittel mit Zitronensäure als viruzidem, bakterizidem und sporizidem Wirkstoff dienen zur Inaktivierung von Hepatitis-B-viren, von bakteriellen Sporen und von Legionella pneumophila bei der Desinfektion von insbesondere thermolabilen medizinischen Instrumenten. Aber auch dieses Verfahren gibt Anlaß, nach weiteren Verbesserungen zu suchen. Die Verbesserung der bekannten Verfahren zur Desinfektion von medizinischen Instrumenten und Geräten war auch Aufgabe der im folgenden beschriebenen Erfindung.

Gegenstand der US-A-5,043,357 ist ein viruzides Mittel, enthaltend mindestens 70 Gew.-% Ethanol und/oder Propanol sowie 0,5 bis 5 Gew.-% einer kurzkettigen organischen Säure. Als Beispiele solcher Säuren werden genannt: Glykolsäure, Zitronensäure, Milchsäure, Bemsteinsäure, Äpfelsäure und Cyclohexylsulfaminsäure. Derartige Mittel finden Verwendung als Händedesinfektionsmittel.

Gegenstand der Erfindung ist ein Verfahren zur Desinfektion von medizinischen Instrumenten und Geräten, dadurch gekennzeichnet, daß diese Instrumente und Geräte bei einer Temperatur oberhalb von 50 °C mit einer wäßrigen Lösung behandelt werden, die Glykolsäure enthält.

Das erfindungsgemäße Verfahren zeichnet sich vor allem dadurch aus, daß neben der eigentlichen desinfizierenden Wirkung auch eine überraschend hohe Reinigungswirkung zu beobachten ist, so daß in den meisten Fällen auf getrennte Reinigungsschritte vollständig verzichtet werden kann. Darüber hinaus wurde überraschenderweise festgestellt, daß mit dem erfindungsgemäßen Verfahren nicht nur Mikroorganismen abgetötet und Viren zerstört werden, sondern daß auch Endotoxine, die aus Mikroorganismen stammen, in deutlichem Maße zerstört werden. Endotoxine können starke Abwehrreaktionen im Organismus verursachen, die ganz unabhängig von einer Infektion sein können und die von Kopfschmerzen über Schüttelfrost, Fieberschübe bis zur Störung des Glukose-Stoffwechsels und zu Veränderungen an den Nebennieren reichen können. Weiterhin ist vorteilhaft, daß das neue Verfahren praktisch keine Korrosion an Edelstahlteilen verursacht und daß die Abwasserbelastung, gemessen als chemischer Sauerstoffbedarf (CSB), und das Risiko zur Bildung von schwerlöslichen Kalziumsalzen im Abwasserkanal gegenüber vergleichbaren Verfahren deutlich geringer sind.

Besonders hervorzuheben ist die ausgezeichnete Wirkung der wäßrigen Glykolsäurelösungen bei erhöhter Temperatur auch gegen Bakteriensporen. Diese abtötende Wirkung auf Sporen ist bei der Desinfektion medizinischer Instrumente besonders notwendig, wird aber von zahlreichen Desinfektionswirkstoffen, die gegen lebende Bakterien wirken, nicht oder nicht in ausreichendem Maße erbracht. Die Verwendung von Glykolsäure in wäßriger Lösung bei erhöhter Temperatur zur Inaktivierung von Bakteriensporen an medizinischen Instrumenten und Geräten ist daher ein besonderer Gegenstand der Erfindung.

Die Vorteile des erfindungsgemäßen Verfahrens beruhen ganz wesentlich auf der Verwendung von Glykolsäure als dem für die Reinigung und Desinfektion verantwortlichen Agens. Die Reinigungswirkung, selbst von stark verdünnten Glykolsäurelösungen, gegenüber Eiweiß-, Blut- und Fettverschmutzungen der Instrumente und Geräte ist überraschenderweise so ausgeprägt, daß in den meisten Fällen auf eine Vorreinigung oder Nachreinigung verzichtet werden kann. Ebenso ist es in der Regel nicht notwendig, den Desinfektionslösungen weitere reinigungsaktive Bestandteile, beispielsweise Tenside oder Komplexbildner, zuzusetzen, wenn es auch Sonderfälle geben kann, in denen das vorteilhaft oder zweckmäßig ist.

Glykolsäure ist eine organische Säure, die in der Natur vorkommt, aber auf einfachem Wege auch synthetisch zugänglich ist. Sie ist in reiner kristalliner Form erhältlich oder aber als wäßrige Lösung, wobei Glykolsäure in wäßriger Lösung je nach Konzentration im Gleichgewicht mit ihrem Autokondensationsprodukt, dem sogenannten Polyglykolid, steht. Soweit im folgenden Konzentrationsangaben gemacht werden, umfassen diese Angaben sowohl die Konzentration an Glykolsäure als auch die Glykolsäureäquivalente aus gegebenenfalls enthaltenem Polyglykolid. Glykolsäure weist nur eine geringe Toxizität auf und ist biologisch mit geringem Sauerstoffbedarf hervorragend abbaubar.

In den Lösungen, die in dem erfindungsgemäßen Verfahren zur Desinfektion der Instrumente und Geräte unmittelbar eingesetzt werden, kann je nach Bedingungen mit sehr geringen Glykolsäurekonzentrationen eine ausreichende Reinigung und Desinfektion erreicht werden. Sowohl die Reinigungs- als auch die Desinfektionswirkung hängt von der Temperatur und von der Einwirkzeit ab. Üblicherweise kann beim erfindungsgemäßen Verfahren mit Glykolsäurekonzentrationen in dieser Lösung von etwa 0,05 bis etwa 2 Gew.-% gearbeitet werden, doch können auch diese Werte in Einzelfällen noch unter- bzw. überschritten werden. Ein bevorzugter Konzentrationsbereich liegt zwischen 0,1 und 1 Gew.-%, insbesondere zwischen 0,4 und 0,7 Gew.-% Glykolsäure in der Anwendungslösung. Die Verwendung zur Inaktivierung von Sporen erfordert keine höheren Konzentrationen.

Die Temperaturen, bei denen die Anwendungslösung in dem erfindungsgemäßen Verfahren auf die Instrumente oder Geräte einwirkt, liegt in jedem Fall deutlich oberhalb der Temperaturen, wie sie in sogenannten manuellen Desinfektionsverfahren angewendet werden, d.h. deutlich oberhalb von 40 °C. Besonders bevorzugt werden Temperaturen ab 60 °C, wobei der Temperaturbereich zwischen etwa 70 und etwa 90 °C noch stärker bevorzugt wird. Wegen des Siedepunkts von Wasser stellt die Temperatur von 100 °C eine praktische Obergrenze für das erfindungsgemäße Verfahren dar, sofern in Sonderfällen nicht unter Druck gearbeitet wird.

Die Einwirkzeit der Glykolsäurelösung auf die Instrumente oder Geräte kann, ohne die desinfizierende oder reinigende Wirkung des Verfahrens zu beeinträchtigen, in weiten Grenzen den praktischen Notwendigkeiten angepaßt werden, wenn die beiden anderen Parameter, nämlich die Glykolsäurekonzentration und die Temperatur entsprechend gewählt werden. Die Einwirkzeiten liegen üblicherweise im Minutenbereich, wobei aber in Sonderfällen die Glykolsäurelösung auch mehrere Stunden auf die Instrumente oder Geräte einwirken darf. Bevorzugte Einwirkzeiten bei mittleren Konzentrationen und Temperaturen liegen zwischen 5 und 20 Minuten, insbesondere zwischen etwa 8 und etwa 15 Minuten. In Sonderfällen, wenn etwa die anfänglich hohe Glykolsäurekonzentration in der Anwendungslösung durch apparatebedingte Verdünnung im Laufe der Einwirkungszeit verdünnt wird, bestimmt sich die Einwirkzeit danach, wie lange noch eine zunehmende Desinfektionswirkung zu beobachten ist. Die Verwendung zur Inaktivierung von Sporen erfordert keine verschärften Bedingungen.

Im einfachsten Falle werden erfindungsgemäß Desinfektionslösungen verwendet, die außer Glykolsäure und Wasser keine weiteren Bestandteile enthalten. In Einzelfällen, wenn dies aus besonderen Gründen gewünscht wird, kann die Desinfektionslösung aber weitere Hilfs- und Zusatzstoffe enthalten. Zu erwähnen sind hier in erster Linie Korrosionsinhibitoren, Netzmittel, Tenside, Komplexbildner, Farbstoffe und ggf. wassermischbare organische Lösungsmittel. Beispiele derartiger Hilfs- und Zusatzstoffe sind anionische Tenside, wie Alkylbenzolsulfonat, Alkylsulfate, Alkyl-Ether-Sulfate und nichtionische Tenside, wie Additionsprodukte von Ethylenoxid an langkettige Verbindungen, insbesondere aus den Klassen der Alkohole, Fettsäuren und Fettsäureamide, wobei ein Teil des Ethylenoxids auch durch Propylenoxid ersetzt sein kann. Im allgemeinen werden schaumarme Tenside bevorzugt. Als weitere Beispiele seien Phosphonobutantricarbonsäure, Natriumbenzoat und Natriumcumolsulfonat genannt.

Die Konzentration an Hilfs- und Zusatzstoffen richtet sich nach dem gewünschten Effekt und kann je nach Wirkstoff sehr unterschiedlich sein. Selbstverständlich soll bei der Wahl der Hilfs- und Zusatzstoffe und ihrer Konzentrationen darauf geachtet werden, daß die positiven Wirkungen der Glykolsäure nicht beeinträchtigt werden.

Die Herstellung der erfindungsgemäß verwendeten Desinfektionslösung geschieht im einfachsten Falle durch getrennte oder gemeinsame Auflösung der Wirk-, Hilfs- und Zusatzstoffe in Wasser, doch ist es im allgemeinen zweckmäßiger, die Anwendungslösung durch entsprechende Verdünnung aus einer konzentrierten Glykolsäurelösung, die ggf. weitere Hilfs- und Zusatzstoffe in der notwendigen Menge enthält, herzustellen. Besonders bevorzugte Konzentrate enthalten etwa 5 bis etwa 40 Gew.-%, insbesondere etwa 15 bis etwa 30 Gew.-% an Glykolsäure.

Zur Desinfektion der Instrumente und Geräte wird entweder die auf Anwendungskonzentration eingestellte Glykolsäurelösung zunächst erhitzt und dann mit den Instrumenten oder Geräten in Kontakt gebracht, beispielsweise durch Einlegen der Instrumente oder durch Einfüllen der Lösung in die Geräte, oder aber es werden die Instrumente zunächst in die Lösung eingelegt oder die Geräte damit befüllt und die Lösung anschließend auf geeignetem Wege mit den Instrumenten und in den Geräten erhitzt. Besonders gebräuchlich ist die Anwendung der Lösung in dafür vorgesehenen Maschinen, in denen die Lösung dann während des Gebrauchs auf die entsprechende Temperatur aufgeheizt und für die notwendige Zeit dort gehalten wird. Geräte, deren Innenraum desinfiziert werden soll, wie beispielsweise Hämodialysegeräte, für die sich das erfindungsgemäße Verfahren besonders gut eignet, besitzen in der Regel eigene Desinfektionsprogramme, mit denen die Aufheizung und gegebenenfalls die Umwälzung der Desinfektionslösung zeitlich gesteuert wird und bei denen ggf. auch schon die Herstellung der Desinfektionslösung durch Verdünnung eines geeigneten Konzentrats über ein Dosierprogramm gesteuert wird. Über die Wahl der richtigen Glykolsäurekonzentration im Konzentrat kann das Verfahren an die bei den einzelnen Maschinen verfügbaren Programme mit ihren Temperatur- und Zeitvorgaben angepaßt werden. Nach der Durchführung des erfindungsgemäßen Reinigungs- und Desinfektionsverfahrens werden die Instrumente und Geräte in der Regel mit Wasser gespült, um sie von der Desinfektionslösung und den abgelösten Verschmutzungen zu befreien. Bei Geräten, die den Reinigungsvorgang selbst steuern, wird in der Regel auch das Nachspülen und ggf. das Trocknen vom Gerät gesteuert.

Nur sofern in Sonderfällen die Reinigungs- oder Desinfektionswirkung des erfindungsgemäßen Verfahrens noch erweitert werden soll, kann es zweckmäßig sein, die Geräte oder Instrumente von Zeit zu Zeit oder gar abwechselnd auch einem anderen Reinigungs- und/oder Desinfektionsverfahren zu unterwerfen, das ergänzende Wirkungsschwerpunkte aufweist. Bei Hämodialysegeräten kann beispielsweise eine gelegentliche zusätzliche Reinigung mit hochalkalischen Mitteln bei starker Fettverschmutzung und zur weiteren Desaktivierung der Endotoxine angezeigt sein. Ein weiterer Gegenstand der Erfindung ist daher ein kombiniertes Aufbereitungsprogramm für Hämodialysegeräte, in dem im Wechsel mit der chemothermischen Desinfektion durch eine Glykolsäurelösung eine Behandlung mit einer hochalkalischen wäßrigen Lösung bei erhöhter Temperatur durchgeführt wird, wobei diese Lösung insbesondere Wirkstoffe aus der Gruppe Alkalihydroxide, Alkaliphosphate, Alkalisilikate und deren Mischungen in Mengen von vorzugsweise etwa 0,2 bis etwa 5 Gew.-% enthält. Der pH-Wert dieser Lösung soll in jedem Falle oberhalb von 10, vorzugsweise oberhalb von 11 und insbesondere oberhalb von 12 liegen (gemessen bei 20 °C). Die Einwirkzeiten der alkalischen Lösung liegen vorzugsweise zwischen etwa 5 und etwa 60 Minuten; die Einwirktemperaturen oberhalb von 50 °C, vorzugsweise oberhalb von etwa 60 °C, insbesondere zwischen etwa 70 und etwa 90 °C. In einer besonders bevorzugten Ausführungsform enthält diese alkalische Lösung wenigstens ein Alkalihydroxid und wenigstens eine Substanz aus der Gruppe Alkaliphosphate, Alkalisilikate und Alkalicarbonate.

### Beispiele

### 1. Wirksamkeit von Glykolsäure gegen Bakteriensporen

Die Wirksamkeit der Glykolsäure gegen Bakteriensporen wurde in Anlehnung an die Richtlinien für die Prüfung und Bewertung chemischer Desinfektionsverfahren (Stand 01.01.1981) der Deutschen Geseltschaft für Hygiene und Mikrobiologie (DGHM) im quantitativen Suspensionsversuch geprüft. Die Prüfung erfolgte im Vergleich mit vorbekannten Desinfektionsmitteln auf Basis von Zitronensäure. Die geprüften Desinfektionsmittellösungen hatten folgende Zusammensetzung:
(A) (erfindungsgemäß):
   0,6 Gew.-% Glykolsäure in Wasser
B (Vergleich):
   0,6 Gew.-% eines Gemisches aus Ameisensäure und Zitronensäure (1 : 3) in Wasser
C (Vergleich):
   0,6 Gew.-% eines Gemisches aus Zitronensäure, Äpfelsäure und Milchsäure (9 : 1 : 1) in Wasser

Zur Prüfung wurden 0,1 ml einer Suspension von Bacillus subtilis Sporen (Lieferant Firma Merck) mit 10 ml der zu prüfenden Desinfektionsmittellösung, die auf 60 °C erwärmt war, gut vermischt. Nach 30 Minuten, 60 Minuten und 120 Minuten bei dieser Temperatur wurde jeweils 1 ml entnommen und mit 9 ml Inaktivierungsflüssigkeit vermischt. Diese Mischungen wurden weiter verdünnt und von den einzelnen Verdünnungsstufen je 0,1 ml auf je 3 Caseinpepton-Sojabohnenmehlpepton-Agar-Platten ausgespatelt. Nach Bebrüten dieser Subkulturen über 48 Stunden bei 37 °C wurde die Anzahl der überlebenden Sporen anhand der gebildeten Bakterienkolonien auf den Platten ausgezählt. Die folgende Aufstellung gibt in logarithmischen Werten die gemittelten Reduktionsfaktoren wieder, die mit den Desinfektionsmittellösungen gegenüber der Einwirkung von reinem Wasser bei dieser Temperatur gefunden wurden. Es ist deutlich zu erkennen, daß bei Einwirkung von Glykolsäure etwa 3 bis 10 mal weniger Sporen überlebten als bei Einwirkung der Desinfektionsmittel auf Basis Zitronensäure. Bei der Behandlung mit Wasser allein bei 60 °C war praktisch keine Abnahme der Sporenzahlen zu bemerken.

| **Desinfektionslösung** | **30 min** | **60 min** | **120 min** |
|---|---|---|---|
| A | 1,22 | 1,45 | 1,54 |
| B | 0,17 | 0,51 | 1,0 |
| C | 0,26 | 0,71 | 1,09 |

### 2. Desinfektion von Hämodialysegeräten

Die desinfizierende Wirkung des erfindungsgemäßen Verfahrens wurde an einem Hämodialysegerät vom Typ Fresenius A 2008 C im Vergleich zu einem herkömmlichen Desinfektionsreiniger auf Basis Kalilauge geprüft. Dabei wurde im Heißdesinfektionsprogramm dieses Gerätes gearbeitet, das in einer Aufheizphase von ca. 15 Minuten auf 85 °C und einer Haltezeit bei dieser Temperatur von etwa 20 Minuten besteht. Die Dosierung der Desinfektions- und Reinigungsmittelkonzentrate erfolgte über eine Zusatzpatrone, die an diesem Gerät üblicherweise der Säurebehandlung nach Bicarbonateinsatz dient.

Im erfindungsgemäßen Verfahren wurde eine wäßrige Lösung von Glykolsäure mit einem Gehalt von 20 Gew.-% verwendet. Der im Vergleichsverfahren verwendete desinfizierende Reiniger enthielt 20 Gew.-% Pentakaliumtriphosphat, 9 Gew.-% KOH und 6 Gew.-% Kaliwasserglas in wäßriger Lösung. Diese Konzentrate wurden von der Maschine automatisch im Verhältnis 1 : 34 während des Aufheizens verdünnt. Als Testkeim diente Bacillus subtilis in Sporenform (ATCC 6633, in Ampullen, Firma Serva).

### 2.1. Versuchsdurchführung

Der Inhalt einer Ampulle der Sporensuspension (Inhalt 1 ml) wurde 500 ml Leitungswasser zugesetzt. Mit Hilfe des Programmes "chemische Desinfektion" bei 37 °C wurde das Gerät mit 200 ml dieser Lösung kontaminiert. Nach einer 15minütigen Verweildauer im Gerät folgte das Programm "Spülung" mit einer Dauer von ebenfalls ca. 15 Minuten. Die dann folgende "Heißdesinfektion" in Kombination mit Desinfektionsmittel dauerte wie oben beschrieben, etwa 35 Minuten. Nach Abschluß der "Heißdesinfektion" schloß sich zur Abkühlung des Gerätes nochmals eine ca. 5minütige Spülung an.

Mit Erreichen der Endtemperatur von 85 °C nach ca. 15 Minuten war etwa zeitgleich die Dosierung des Desinfektionsmittels beendet. Da das Gerät die Desinfektionsmittellösung nicht im Kreislauf fährt, sondern während des gesamten Programms "Heißdesinfektion" eine Durchflußrate von ca. 300 ml pro Minute hat, wurde die Soll-Anwendungskonzentration von ca. 3 % im Laufe der sich anschließenden 20minütigen Haltezeit bei 85 °C immer weiter verdünnt.

### Zusammenfassung der Programmschritte:

| Schritt | Programm | Dauer ca. |
|---|---|---|
| 1. Kontamination | "Chemische Desinfektion 37 °C" | 15 Minuten |
| 2. Ruhepause | | 15 Minuten |
| 3. Spülung | "Spülung" | 15 Minuten |
| 4. Desinfektion | "Heißdesinfektion 85 °C" | 35 Minuten |
| 5. Abkühlung | "Spülung" | 5 Minuten |

### 2.2. Probenahme und Ergebnisse:

Zur Kontrolle des Desinfektionserfolges wurden Spülwasserproben und Tupferabstriche entnommen.

### Spülwasserproben:

S1. Kontaminationslösung vor Versuch (vor Schritt 1)
S2. Spülwasser kurz vor Ende Schritt 1 (Kontamination)
S3. Spülwasser kurz vor Ende Schritt 3 (Spülung)
S4. Spülwasser kurz vor Ende Schritt 4 (Desinfektion)
S5. Spülwasser kurz vor Ende Schritt 5 (Spülung)

Die Spülwasserproben, die gegen Ende des Programmes "Heißdesinfektion" entnommen wurden, wurden sofort nach Entnahme auf Raumtemperatur abgekühlt.

### Aufarbeitung:

Je nach der zu erwartenden Keimdichte erfolgte der Keimnachweis durch die Ausplattierung von 0,1 ml aus einer Verdünnungsreihe (Probe 1 und 2) oder durch die Membranfiltration von 1 ml, 10 ml und 100 ml (Proben 3 bis 5). Bei Membranfiltration der Spülwasserprobe Nr. 4 (nach Desinfektion) wurden die Filter zur Entfernung evtl. vorhandener Desinfektionsmittelreste mit je 2 x 50 ml Inaktivatorlösung gespült.

### Bebrütung: 18 Stunden bei 37 °C auf CaSo-Agar

### Ergebnisse:

In der folgenden Tabelle sind die Anzahl der koloniebildenden Einheiten (KBE) in Abhängigkeit vom Versuch und untersuchter Menge von 1, 10 und 100 ml angegeben.

| Spülwasserprobe | Menge in ml | Erfindungsgemäßes Verfahren | Vergleichsverfahren |
|---|---|---|---|
| S1 | 1 | 2,4x10⁵ | 1,8x10⁵ |
| S2 | 1 | 4,9x10⁴ * | 8,3 x 10³ * |
| S3 | 1 | 2,3 x 10¹ | 4,3 x 10¹ |
| | 10 | 2,2 x 10² | 4,0 x 10² |
| | 100 | n.a. | n.a. |
| S4 | 10 | o.B. | 8 |
| | 100 | 8 | 4,2 x 10¹ |
| S5 | 10 | o.B. | 6 |
| | 100 | 6 | 4,7 x 10¹ |

| | | | |
|---|---|---|---|
| * Diese Keimzahl stellt die eigentliche Ausgangskeimzahl des Versuches dar. o.B. = ohne Befund n.a. = nicht auszählbar | | | |

### Tupferproben

T1 Kupplung rot (Anschluß zum Dialysator)
T2 Kupplung blau (Anschluß zum Dialysator)
T3 Schwimmer oben
T4 Schwimmer unter Wasserlinie
T5 Kammerwand (Schwimmerkammerwand)
T6 Gummidichtung (Schwimmerkammer)

### Aufarbeitung:

Die Tupfer wurden auf Caso-Agar ausgestrichen und dann zum Nachweis kleinster Keimgehalt in Caso-Bouillon angereichert.
- Bebrütung:: 18 Stunden bei 37 °C auf CaSo-Agar
48 Stunden bei 37 °C in CaSo-Bouillon

### Ergebnisse:

| Erfindungsgemäßes Verfahren | | Vergleichsverfahren |
|---|---|---|
| Tupfer Nr. | Ausstrich/Anreicherung | Ausstrich/Anreicherung |
| T1 | o.B. / o.B. | o.B. / o.B. |
| T2 | o.B. / o.B. | o.B. / o.B. |
| T3 | o.B. / + | 8 /+ |
| T4 | O.B./+ | 1 /+ |
| T5 | 2 /+ | 39 /+ |
| T6 | - /- | 3 /+ |
| o.B. = ohne Befund (0) + = positiv im Bezug auf Wachstum des Testkeimes - = nicht untersucht | | |

## Patentansprüche

1. Verfahren zur Desinfektion von medizinischen Instrumenten und Geräten, **dadurch gekennzeichnet, daß** diese Instrumente und Geräte bei einer Temperatur oberhalb von 50 °C mit einer wäßrigen Lösung behandelt werden, die Glykolsäure enthält.

2. Verfahren nach Anspruch 1, bei dem die Konzentration an Glykolsäure in der Behandlungslösung zwischen 0,1 und 1 Gewichtsprozent liegt.

3. Verfahren nach Anspruch 2, bei dem die Konzentration an Glykolsäure in der Behandlungslösung zwischen 0,4 und 0,7 Gew.-% liegt.

4. Verfahren nach einem der Ansprüche 1 bis 3, bei dem die Behandlung der Instrumente mit Glykolsäurelösung einer Temperatur oberhalb von 60 °C vorgenommen wird.

5. Verfahren nach einem der Ansprüche 1 bis 4, bei dem die Einwirkzeit der Glykolsäurelösung auf die Instrumente oder Geräte bei der erhöhten Temperatur 5 bis 20 Minuten beträgt.

6. Verfahren nach einem der Ansprüche 1 bis 5, bei dem die Glykolsäurelösung, mit der die Instrumente oder Geräte behandelt werden, durch Verdünnen mit Wasser aus einem Konzentrat hergestellt wird, das die Glykolsäure in der nötigen Menge enthält.

7. Verfahren nach Anspruch 6, bei dem die Glykolsäurelösung durch Verdünnen mit Wasser aus einem Konzentrat hergestellt wird, das neben der Glykolsäure ferner weitere Hilfs- und Zusatzstoffe, ausgewählt aus Korrosionsinhibitoren, Netzmitteln, Tensiden, Komplexbildnern und Farbstoffen, enthält.

8. Verfahren nach einem der Ansprüche 1 bis 7 zur Desinfektion von Hämodialysegeräten.

9. Aufbereitungsverfahren für Hämodialysegeräte, bei dem ein Desinfektionsverfahren nach einem der Ansprüche 1 bis 8 im Wechsel mit einer Behandlung der Geräte mit einer hochalkalischen wäßrigen Lösung bei einer Temperatur oberhalb von 50 °C durchgeführt wird.

10. Verwendung von Glykolsäure in wäßriger Lösung in einem Verfahren gemäß einem der Ansprüche 1 bis 9 zur Inaktivierung von Bakteriensporen.

## Claims

1. A process for disinfecting medical instruments and equipment, **characterized in that** the instruments and equipment are treated at a temperature above 50°C with an aqueous solution containing glycolic acid.

2. A process as claimed in claim 1, **characterized in that** the concentration of glycolic acid in the treatment solution is between 0.1 and 1% by weight.

3. A process as claimed in claim 2, **characterized in that** the concentration of glycolic acid in the treatment solution is between 0.4 and 0.7% by weight.

4. A process as claimed in any of claims 1 to 3, **characterized in that** the treatment of the instruments with glycolic acid solution is carried out at a temperature above 60°C.

5. A process as claimed in any of claims 1 to 4, **characterized in that** the instruments or equipment are exposed to the glycolic acid solution at the elevated temperature for a period of 5 to 20 minutes.

6. A process as claimed in any of claims 1 to 5, **characterized in that** the glycolic acid solution with which the instruments or equipment are treated is prepared from a concentrate containing the glycolic acid in the requisite quantity by dilution with water.

7. A process as claimed in claim 6, **characterized in that** the glycolic acid solution is prepared by dilution with water from a concentrate which, besides the glycolic acid, contains other auxiliaries and additives selected from corrosion inhibitors, wetting agents, surfactants, complexing agents and dyes.

8. A process as claimed in any of claims 1 to 7 for disinfecting haemodialysis equipment.

9. A process for maintaining haemodialysis equipment in which the disinfection process claimed in any of claims 1 to 8 is carried out in alternation with the treatment of the equipment with a highly alkaline aqueous solution at a temperature above 50°C.

10. The use of glycolic acid in aqueous solution in the process claimed in any of claims 1 to 9 for inactivating bacterial spores.

## Revendications

1. Procédé de désinfection d'instruments et d'appareils médicaux,
**caractérisé en ce que**
ces instruments et appareils sont traités à une température au-dessus de 50°C avec une solution aqueuse qui contient de l'acide glycolique.

2. Procédé selon la revendication 1,
dans lequel
la concentration en acide glycolique dans la solution de traitement se situe entre 0.1 et 1 % en poids.

3. Procédé selon la revendication 2,
dans lequel
la concentration en acide glycolique dans la solution de traitement se situe entre 0,4 et 0,7 % en poids.

4. Procédé selon l'une des revendications 1 à 3,
dans lequel
le traitement des instruments avec la solution d'acide glycolique s'effectue à une température au-dessus de 60°C.

5. Procédé selon l'une des revendications 1 à 4,
dans lequel le temps d'action de la solution d'acide glycolique sur les instruments et sur les appareils à la température accrue est de 5 à 20 minutes.

6. Procédé selon l'une des revendications 1 à 5,
dans lequel
la solution d'acide glycolique avec laquelle on a traité les instruments ou les appareils, est préparée par dilution avec de l'eau à partir d'un concentré qui contient l'acide glycolique dans la quantité requise.

7. Procédé selon la revendication 6,
dans lequel
la solution d'acide glycolique est préparée par dilution avec de l'eau à partir d'un concentré qui contient à côté de l'acide glycolique en outre d'autres adjuvants et additifs choisis parmi les inhibiteurs de corrosion, les agents humidifiants, les agents tensioactifs, les agents complexants et les colorants.

8. Procédé selon l'une des revendications 1 à 7,
en vue de la désinfection des appareils d'hémodialyse.

9. Procédé de traitement pour appareils de dialyse,
dans lequel
on effectue un processus de désinfection selon l'une des revendications 1 à 8, en changement avec un traitement des appareils avec une solution aqueuse fortement alcaline à une température au-dessus de 50°C.

10. Utilisation de l'acide glycolique en solution aqueuse dans un procédé selon l'une des revendications 1 à 9,
en vue de l'inactivation de spores bactériennes.
